# DEMANDE DE BREVET EUROPEEN

(11) **EP 1 434 052 A1**
(43) Date de publication de la demande: **30.06.2004**
(21) Numéro de dépôt: 03293250.1
(22) Date de dépôt: 19.12.2003
(51) Int. Cl.: G01N 33/50, G01N 21/76

(54) **Procédé d'évaluation de l'activité cosmétique ou dermatologique d'un produit en utilisant un matériel végétal**

(30) Priorité: 20.12.2002 FR 0216390
(71) Demandeur: L'OREAL, 75008 Paris (FR)
(72) Inventeur: Lefebvre, Marc André, 86600 Coulombiers (FR); Martin, Richard, 37210 Rochecorbon (FR); Kauffmann, Myriam, 75014 Paris (FR)
(74) Mandataire: Allab, Myriam

(57) **Abrégé**

La présente invention concerne un procédé d'évaluation de l'activité cosmétique ou dermatologique d'un produit caractérisé en ce qu'il comprend une étape consistant à mettre en contact le produit à évaluer avec un matériel végétal et à déterminer la variation d'un paramètre mécanique, optique ou physico-chimique dudit matériel végétal dans la zone de contact avec le produit, et/ou induite par le produit à évaluer. Elle concerne également un procédé de criblage d'actifs et l'utilisation d'un matériel végétal ou d'un fragment de celui-ci pour évaluer l'activité cosmétique ou dermatologique d'un produit.

## Description

La présente invention se rapporte à des procédés d'évaluation ou de criblage de l'activité d'un produit dans les domaines cosmétique ou dermatologique et à l'utilisation d'un nouveau modèle pour évaluer l'efficacité et/ou les propriétés organoleptiques d'un tel produit.

Pour déterminer l'activité d'une molécule ou d'un produit, ou sélectionner des molécules ou produits actifs parmi un groupe de candidats potentiels, la mise en oeuvre de tests in vivo est la voie donnant les résultats les mieux extrapolables à une application à grande échelle à l'homme. Cependant, ce type d'expérimentation ne peut être effectué chez l'être humain en première intention, puisque le produit à tester doit avoir été reconnu sans danger tant sur le plan toxicologique qu'immunologique; cette phase implique en outre des précautions réglementaires et une information des sujets qui ralentissent sa mise en oeuvre. L'expérimentation animale, parfois utilisée comme alternative, ne peut pour des raisons éthiques, s'y substituer. En outre, la cible cosmétique n'est pas toujours transposable dans ce modèle, notamment dans la mesure où elle s'intéresse à une perception.

On utilise classiquement des tests in vitro pour objectiver l'activité d'un produit nouveau, ou une nouvelle activité d'un produit connu. Il s'agit généralement de cultures cellulaires qui miment des mécanismes responsables, in vivo, des activités ciblées. L'extrapolation à l'homme de ces résultats peut parfois être difficile. De plus, leur analyse nécessite des techniques très performantes et très sensibles lorsque l'on souhaite objectiver des effets cosmétiques, dont le seuil d'activité est faible ou l'apparition des conséquences lentes.

Les modèles sur peau reconstituée donnent de bons résultats, toutefois il s'agit de techniques qui peuvent être délicates à mettre en oeuvre.

Il serait donc souhaitable de disposer d'une phase d'évaluation précoce sur un modèle vivant, pouvant être standardisé, et n'impliquant pas les contraintes d'une phase toxicologique ou clinique. Il serait également souhaitable de disposer d'un complément aux approches *in vitro* et *in vivo* chez l'homme, et permettant de mieux prévoir les activités in vivo intrinsèques des produits ou actifs envisagés.

De manière inattendue, la demanderesse a pu montrer que ces buts et d'autres sont atteints par l'utilisation d'un modèle végétal selon l'invention.

C'est pourquoi la présente invention a pour objet un procédé d'évaluation de l'activité cosmétique ou dermatologique d'un produit, caractérisé en ce qu'il comprend une étape consistant à mettre en contact le produit à évaluer avec un matériel végétal et à déterminer la variation d'un paramètre mécanique, comportemental, optique ou physico-chimique dudit matériel végétal, induite par le produit à évaluer. Ledit paramètre pourra être biologique.

La variation induite par le produit peut être déterminée selon l'invention dans la zone de contact du matériel végétal avec le produit à évaluer, et/ou dans une zone distante de la zone de contact ou d'application dudit produit.

En effet, les plantes sont des organismes vivants, d'une très grande diversité, qui peuvent mimer un grand nombre d'états de la peau ou des phanères. Le matériel végétal pourra notamment servir de modèle pour évaluer des états de surface de la peau, des réactions métaboliques internes telles que des réactions enzymatiques, des activités de secrétion et/ou des échanges gazeux.

La plante peut ainsi reproduire par exemple les mécanismes de défense mis en jeu par la peau, qu'il s'agisse de l'effet barrière, de l'hydratation ou de la desquamation, de la lutte contre l'oxydation, la pollution ou les radiations lumineuses. Elle peut également mimer la transmission d'information de nature sensitive.

Par produit, on entend aussi bien un ingrédient ou un actif sous une forme plus ou moins purifiée, présentant une activité intrinsèque *in vitro* ou *in vivo*, qu'une formulation comprenant un ou plusieurs de ces ingrédients et un support et des adjuvants adaptés à l'application visée.

Ce procédé est particulièrement adapté à l'évaluation de produits cosmétiques.

Par "produit cosmétique", on entend notamment toute substance ou préparation destinée à être mise en contact avec les diverses parties superficielles du corps humain (épiderme, système pileux et capillaire, ongles, lèvres et organes génitaux externes) ou avec les dents et les muqueuses buccales en vue, exclusivement ou principalement, de les nettoyer, de les parfumer, d'en modifier l'aspect et/ou de corriger les odeurs corporelles et/ou de les protéger ou de les maintenir en bon état (directive cosmétique 76/768/CEE modifiée).

Cependant, au sens de la présente invention on entend également par produits cosmétiques des actifs ou des compositions destinés à être absorbés par toute voie permettant un passage systémique, en particulier par voie orale, en vue de protéger ou maintenir en bon état les parties superficielles du corps, ou d'améliorer l'apparence des individus, en particulier au niveau de la peau et ses annexes.

A titre d'exemples non limitatifs de produits cosmétiques, on peut citer tout produit destiné à réduire les signes du vieillissement de la peau ou du cheveu, notamment les rides, à hydrater la peau, à nettoyer et/ou à nourrir et/ou à entretenir la peau ou les cheveux, à désodoriser la peau, à la préparer à une exposition solaire, à en renforcer l'élasticité, à en améliorer la douceur, etc..

Les activités cosmétiques que l'on peut évaluer selon l'invention seront notamment choisies dans le groupe comprenant les effets sur l'hydratation de la peau, l'effet anti-rides, l'amélioration de la fonction barrière, la protection vis-à-vis des rayonnements solaires, l'amélioration des propriétés mécaniques de la peau, les effets tenseurs, la desquamation, la pigmentation des cheveux ou de la peau, l'effet d'interaction avec les odeurs ou leur modification, notamment atténuation, transformation ou capture, en particulier l'effet désodorisant ou déodorant mais aussi l'effet exhausteur de parfum, l'effet apaisant ou anti-irritant, les propriétés couvrantes, les propriétés optiques colorantes ou masquantes et la tenue du produit.

La demanderesse a montré que ce procédé d'évaluation peut être mis en oeuvre de façon reproductible, avec un bon facteur prédictif. De plus le matériel végétal est un organisme vivant, ayant sa physiologie et il est soumis à l'environnement, comme le revêtement cutané ou les annexes chez l'homme, ce qui permet de reproduire plusieurs conditions auxquelles sera soumis un organisme humain lors de l'application des produits à tester.
Le matériel végétal utile selon l'invention sera avantageusement issu d'un organisme supérieur, à cellules eucaryotes; il s'agit en particulier d'organismes photosynthétiques. Les paramètres mesurés sur ces matériels sont de préférence des facteurs propres à la plante utilisée pour la mise en oeuvre du procédé selon l'invention, et ne seront pas le résultat de l'introduction d'une nouvelle caractéristique par modification génétique.

Le procédé selon l'invention peut être mis en oeuvre sur un organisme végétal dans son entier, ou sur des fragments de celui-ci. On peut notamment utiliser selon l'invention la plante entière, les fleurs, les feuilles, les pétales, les tiges, les fruits, les graines, les racines, entier et/ou leurs fragments. Lorsqu'il s'agit d'un fruit, on peut pour l'évaluation de certaines propriétés, ôter le revêtement cireux présent sur certaines variétés, ou retirer la peau pour favoriser les échanges gazeux.

Le matériel végétal pourra être issu de culture *in vivo* ou *in vitro*. Par culture *in vivo* on entend toute culture de type classique c'est à dire en sol à l'air libre ou en serre, ou encore hors sol.
Par culture *in vitro*, on entend l'ensemble des techniques connues de l'homme du métier qui permet de manière artificielle l'obtention d'un végétal ou d'une partie d'un végétal. La pression de sélection imposée par les conditions physico-chimiques lors de la croissance des cellules végétales in vitro permet d'obtenir un matériel végétal standardisé et disponible tout au long de l'année contrairement aux plantes cultivées *in vivo*.

Selon un mode de réalisation avantageux de l'invention, le matériel végétal sera produit à partir de cellules dédifférenciées ou de plantules obtenues par micro-propagation in vitro.
Par cellules végétales dédifférenciées, on entend toute cellule végétale ne présentant aucun des caractères d'une spécialisation particulière et capable de vivre par elle-même et non en dépendance avec d'autres cellules. Ces cellules végétales dédifférenciées sont éventuellement aptes, sous l'effet d'une induction, à toute différenciation conforme à leur génome (embryons somatiques, cultures racinaires...).
Selon la méthode de culture choisie, et en particulier selon le milieu de culture choisi, il est possible d'obtenir, à partir d'un même explant, des cellules végétales dédifférenciées présentant des caractères différents.

Selon l'un des modes de réalisation de l'invention, le produit à évaluer est mis en contact avec au moins une partie d'un végétal cultivé in vivo, obtenu à partir de matériel végétal ayant subi une étape de culture ou de propagation in vitro.

Le contact pourra être réalisé par application topique du produit à tester. Cette application sur au moins une portion de la surface externe du végétal sera effectuée par tout moyen connu de l'homme du métier et adapté à la forme du produit à tester, tel que par exemple étalement, pulvérisation, trempage, injection, scarification ou mise en contact sous occlusion.

Selon un mode de mise en oeuvre particulier de l'invention, une partie du matériel végétal sera traitée par le produit à tester, éventuellement dans un excipient; une autre partie du même matériel végétal ne sera pas traitée, ou uniquement par l'excipient, et servira de témoin pour la détermination de la variation.

Selon un autre mode de réalisation de l'invention, le produit sera mis en contact avec le matériel végétal sous forme d'un nutriment qui est absorbé par ledit végétal. Les plantes poussent sur des milieux nutritifs supplémentés en l'actif à tester qui sera incorporé par voie systémique. Les effets sur les couches externes de la plante pourront être évalués comme précédemment, mimant alors les phénomènes observés en cosmétique orale. Des actifs pouvant notamment être évalués par cette voie sont des vitamines et/ou des oligoéléments, ou des composants agissant sur la flore microbienne.

Dans tous les cas, l'application pourra être unique ou bien répétée dans le temps, par exemple à intervalles réguliers pendant plusieurs heures, plusieurs jours ou plusieurs semaines.

Selon une variante préférée de l'invention, les conditions auxquelles est soumis le matériel végétal sont contrôlées et peuvent subir des modifications. Ces conditions incluent des paramètres environnementaux comme la luminosité, le rayonnement solaire, la température, la composition en gaz ou en radicaux libres, la présence de particules polluantes, le degré d'hygrométrie.

On mime ainsi différents facteurs tels que l'exposition solaire, le stress ou la pollution, qui peuvent influencer les caractéristiques cosmétiques d'un produit, ou qui sont justement ceux vis-à-vis desquels l'efficacité du produit doit être évaluée.

Toute plante ou fragment de plante pourra être utilisé dans le procédé selon l'invention. Avantageusement, le choix du modèle sera adapté à l'activité cosmétique ou dermatologique que l'on veut évaluer. On peut citer de manière non limitative les plantes de la famille des cactées et de manière générale les plantes désertiques et halophiles, qui seront particulièrement adaptées à l'étude du stress hydrique. Des plantes de haute montagne comme Leontopodium alpinum seront de préférence utilisées pour évaluer une activité vis-à vis d'un stress UV; des fruits rouges pourront également être utilisés pour évaluer un effet protecteur des rayonnements UV. Des modèles adaptés à la mesure de l'hydratation ou de l'effet barrière d'un produit sont par exemple des tubercules, des fruits ou des plantes entières, parmi lesquels on peut citer de manière non limitative *Solanum tuberosum* , *Malus sp., Daucus carota, Ficus benjamina, Syngonium sp.* ou *Spatyphillum sp*.

La variation du paramètre mécanique, optique, physique ou chimique, notamment biochimique, pourra être déterminée selon l'invention par toute méthode qualitative ou quantitative connue de l'homme du métier.

Le matériel végétal peut notamment être observé par des moyens optiques ou spectroscopiques, évalué par des moyens instrumentaux, et/ou analysé avec les mêmes techniques que celles proposées en bioanalyse pour les prélèvements de stratum corneum, de muqueuse, de cuticule de cheveu ou de surface d'ongle ou des sécrétions qui les recouvrent.

La variation des paramètres optique, mécanique et/ou physico-chimique peut notamment être déterminée à l'aide des techniques utilisées classiquement en Evaluation cosmétique : cornéomètre, torquemètre, colorimètre, mesure du pH, sébumètre, lipomètre, microscope confocal à lumière blanche ou microscope confocal à laser, desquamation, épaisseur de l'enveloppe cornéocytaire, microcirculation, et éventuellement par des mesures d'ions ou de potentiels électriques voire même des techniques d'imagerie médicale telles que échographie, IRM...), perte insensible en eau, mesure du plissement cutané (densiscore ), observation microscopique optique ou photonique, prise de photographie, mesure de spectres ( UV, Visible, IR, Raman, Fluorescence ), notamment spectrophotométrie ou spectroradiométrie, analyse chromatographique d'échantillons représentatifs, notamment chromatographie gazeuse, empreinte sur matériaux organique ( silflo ) ou inorganique ( céramique ).
La mesure de spectre de Raman de surface des feuilles pourra notamment être effectuée par microscopie confocale, après irradiation UV, pour des feuilles ayant ou non été soumise à l'application de différents produits à évaluer.

Les paramètres physico-chimiques ou biologiques seront de manière générale mesurés par toute méthode de dosage adaptée connue de l'homme du métier, ou par toute technique de génomique, transcriptomique ou protéomique.

Selon un mode de réalisation du procédé de l'invention, le paramètre mesuré ou évalué pour le matériel végétal est le même que celui qui signerait l'activité du produit chez l'homme. En effet, dans un certain nombre de cas, on observe, après contact avec le produit et/ou exposition à des conditions environnementales, une évolution des facteurs mécaniques et/ou physico-chimiques similaire, bien que d'une amplitude différente, chez l'homme et chez le végétal.

Selon un autre mode de réalisation du procédé de l'invention, le paramètre mesuré ou évalué pour le matériel végétal est différent de celui qui signerait l'activité du produit chez l'homme, mais son évolution est corrélée à une activité établie chez l'homme. A cet égard, on peut citer des mécanismes de réaction aux différents rayonnements lumineux.

La simple observation de l'aspect du matériel végétal permet d'accéder aux paramètres tels que , par exemple, l'éclat ou la bonne santé et permet d'établir des atlas permettant d'améliorer la mesure de ces paramètres de type "clinique".

Les diverses observations optiques photographie ou vidéo mettent en évidence les variations du micro-relief de surface.

Les paramètres d'hydratation sont mesurés de manière connue en soi à l'aide d'un cornéomètre.
Les propriétés mécaniques de la surface protectrice, telles que la fermeté ou l'élasticité, essentielles au rôle du stratum corneum seront par exemple déterminées de la même façon sur le matériel végétal par torquemètrie dermique.

La colorimètrie permettra d'évaluer finement les modifications de couleur du matériel végétal, et les effets protecteurs d'un produit comme par exemple un filtre solaire, ou des anti-oxydants; inversement, cette détermination pourra permettre d'apprécier le pouvoir desquamant d'un actif, ou détergent d'un produit de toilette.
Cette technique pourra également servir à apprécier les propriétés cosmétiques de produits de maquillage, tels que fond de teint, vernis à ongle, rouge à lèvres ou mascaras ou produit de coiffage.

Un modèle particulièrement intéressant à cet égard est représenté par les graines telles que les marrons d'Inde, issus d'*Aesculus hyppocastanum*. Ce modèle est particulièrement adapté à l'étude de produits appliqués à la surface de l'ongle dont il mime :
- la forme bombée,
- la texture de sa surface, brillante proche de la brillance naturelle de l'ongle (donc de son état de surface en termes de micro relief et de porosité) sur la partie marron, la partie blanchâtre (le hile), moins brillante mimant l'état de surface des ongles naturellement moins brillants ou après abrasion par une lime ou un micro ponçage par un instrument de manucure,
- sa couleur: la partie claire arrondie ressemble à la lunule, le passage de la partie claire à la partie marron foncée, arrondie dans le sens convexe, mime le passage de la lunule au plateau unguéal. Le passage beige/marron équivaut aussi à celui du plateau au bord libre de l'ongle en termes de contraste de couleurs bien que la concavité soit inversée.
   Ces couleurs sont particulièrement proches de celles des ongles des sujets noirs ou de carnation foncée.
- les hétérogénéités du relief: la partie beige centrale est souvent encaissée de quelques centaines de micromètres, ce qui est le même ordre de grandeur que les irrégularités observables sur des ongles sains (comme les cannelures des ongles âgés ou une marque de rupture du rythme de croissance)
   Des modèles de châtaignes, fruits de *Castanea sativa*, qui reproduisent ces cannelures, sont également bien adaptés à ce type d'évaluation.

Ces graines sont donc des supports très facile à mettre en oeuvre et bien représentatifs pour tester les propriétés optiques des vernis à ongles (le résultat de maquillage en termes de couleur, reflets 3D sur surface bombée, brillance sur un support organique plus ou moins mat, couvrance de la couleur, couvrance des défauts ou de l'hétérogénéités des ongles), ce qui est la finalité majeure des vernis de maquillage.

Il est possible d'étudier l'effet d'une base (par exemple base lissante ou base camouflante), de la superposition de plusieurs couches,
L'applications de vernis est facile et rapide. Le vernis tient bien. Il peut être démaquillé au dissolvant pour vernis à ongles et remaquillé.
L'effet général est particulièrement intéressant pour juger de l'effet maquillant des formules sur les ongles des sujets à carnation foncée.

Les secrétions à la surface des feuilles et/ou des fruits pourront être mesurées par sébumètrie ou lipomètrie, comme indicateur d'une action sur la secrétion du sébum chez l'homme. Les mesures confocales pourront être effectuées en lumière blanche ou au laser, pour évaluer l'état des couches analogues à celles du stratum corneum.

La mesure de la desquamation pourra être réalisée par recueil des éléments de surface se détachant spontanément ou par grattage calibré par le végétal et comparaison de leur quantité avec celle d'un végétal témoin.

Les variations d'épaisseur de différentes couches protectrices pourront ainsi être mesurées par ultra sons.

Les effets sur la micro-circulation pourront être évalués par le suivi des fluides végétaux mimant grossièrement la lymphe et le sang que sont le phloème (sève élaborée) et le xylème (sève brute). Le xylème circule des racines vers l'apex alors que le phloème circule à contre sens.
Selon une variante du procédé, l'évaluation de l'activité d'un produit sur l'effet barrière comprend les étapes suivantes :
a) application du produit à évaluer sur une portion de matériel végétal
b) contact pendant une durée comprise entre 1 et 10h
c) mesure de la perte insensible en eau (i) sur la portion de matériel végétal ayant été en contact avec le produit à évaluer et (ii) sur une autre portion du même matériel végétal n'ayant pas été en contact avec ledit produit
d) comparaison des résultats des mesures sur les deux portions.

Le prélèvement pourra être effectué par toute méthode connue de l'homme du métier, notamment par la mise en oeuvre du dispositif tel que décrit dans la demande de brevet FR-A-2 667 778, lequel dispositif permet de mettre en circulation un liquide sur la peau en vue de récupérer des éléments présents sur la surface de cette dernière. On peut aussi utiliser les dispositifs décrits dans le brevet FR-A-2 368 708 ou US-A-5 433 214.

Avantageusement le prélèvement sera effectué par un dispositif de mesure et/ou d'analyse d'au moins un paramètre, notamment biologique, mécanique, chimique ou physico-chimique d'une portion externe du corps humain, en particulier de la peau, des ongles ou des cheveux, tel que décrit dans la demande FR0114334. Ce dispositif comprend une surface destinée à être mise en regard ou au contact de ladite portion externe, ledit dispositif étant :
- soit modelable sur ladite portion externe et comprenant un matériau inorganique;
- soit non modelable sur ladite portion externe, et réalisé essentiellement en au moins un matériau inorganique, ladite surface comprenant un matériau inorganique, autre que le verre lorsqu'il n'est pas sous forme fibreuse ou particulaire.

On peut utiliser un tel dispositif pour connaître l'état du stratum corneum, analyser les sécrétions, excrétions, ou les odeurs, présentes à la surface du matériel végétal, ou analyser son contenu bioanalytique, microbiologique ( bactéries, virus, levures, acariens, divers parasites...), ou enzymatique.

Les éléments pouvant être prélevés par le dispositif selon l'invention peuvent être sous forme solide, liquide ou gazeuse (composés volatiles et/ou odorants).

Un tel dispositif peut être également utilisé pour l'évaluation de propriétés mécaniques de revêtement, telles que son élasticité ou des propriétés physiques de surface telles que son relief ou micro-relief, le nombre et/ou la profondeur des rides ou micro-rides ou sillons présentes à sa surface, par la densité et/ou la taille de ses pores ou ostium, ou la douceur ou le glissant.

La mesure de la variation d'un paramètre chimique permettra en particulier de déterminer l'apparition ou la modification d'une réaction biologique ou biochimique, par l'actif ou le produit à tester. On pourra ainsi mesurer l'apparition d'un ou plusieurs métabolites, ou la variations des concentrations en constituants biologiques, substrats ou co-substrats desdites réactions biologiques. Ces dosages concerneront notamment des réactions enzymatiques et pourront par exemple être réalisés par toute méthode enzymatique, immuno-enzymatique, histochimique connue de l'homme du métier.

Selon un mode de réalisation de l'invention, le procédé sera adapté à l'évaluation de l'effet protecteur ou activateur du produit à tester sur une réaction métabolique, dans des conditions ou en présence d'un facteur connu pour influencer ladite réaction métabolique.

L'analyse des gaz dans une enceinte fermée ou une enceinte au contact de la surface du végétal, et notamment les modifications de la teneur en O₂, en CO₂ et/ou H₂O₂, réalisée notamment par chromatographie, permettra d'étudier le métabolisme cellulaire et en particulier les modifications symétriques de celles observées pour le métabolisme cutané lors d'irradiation ou pour l'évaluation d'agents anti-pollution ou anti-radicalaires.

Selon l'un des aspects de l'invention, l'émission d'odeur dans l'environnement peut être suivie par des techniques de chromatographies gazeuse directe sur la plante et notamment la technique de head space qui permet de recueillir les molécules les plus volatiles qui seraient indétectables sur des échantillons conservés. A titre d'exemple les études peuvent être réalisées directement sur l'oignon *(Alium cepa)*, l'ail *(Allium sativum)*, *Euphorbia sp.* ou d'autres parties de plantes alimentaires ou aromatiques dont une partie des molécules émises est proche de celles qui sont excrétées dans la transpiration humaine. Il est possible d'interposer par application ou pulvérisation sur la plante des systèmes de capture d'odeur tels que des cyclodextrines, des zéolites ou divers catalyseurs chimiques pour en évaluer leurs effets.
Il est possible également d'évaluer pour les odeurs agréables telles que celles de fleurs ou de fruits l'effet de compositions destinées à exalter ces odeurs ou à prolonger leur présence à proximité des téguments.

Le procédé d'évaluation selon l'invention peut, selon l'un des modes de réalisation, être utilisé pour cribler un ou plusieurs actifs. En particulier, l'invention a pour objet un procédé de criblage pour identifier des actifs cosmétiques ou dermatologiques caractérisé en ce que l'on
a) évalue l'activité cosmétique ou dermatologique desdits actifs par le procédé tel que défini précédemment,
b) compare les variations des paramètres mécaniques, optiques ou physico-chimiques induites par les actifs à cribler,
c) sélectionne les actifs ayant induits les variations les plus importantes à l'étape b)

Le ou les actifs ainsi sélectionnés seront avantageusement incorporés dans une formulation, dont on validera ainsi la composition. L'invention a donc également pour objet un procédé de préparation d'une composition cosmétique ou dermatologique, caractérisé en ce que l'on
i) sélectionne au moins un actif cosmétique ou dermatologique par le procédé défini ci-dessus,
ii) mélange le ou les actifs sélectionnés à l'étape i) avec des supports physiologiquement et/ou cosmétologiquement acceptables.

Selon un autre aspect, l'invention concerne donc l'utilisation d'au moins un matériel végétal ou d'un fragment de celui-ci pour évaluer l'activité cosmétique ou dermatologique d'un produit.

Un autre avantage de l'invention est la mise en oeuvre d'un modèle végétal sur lequel les consommateurs, et en particulier les consommatrices, pourront se projeter. L'invention a donc également pour objet une méthode pour la promotion d'un produit cosmétique consistant à faire état d'une efficacité, action ou propriété dudit produit démontrée par au moins un test opéré sur un sujet végétal. Cette communication pourra être basée sur le modèle végétal spécifique d'un produit ou d'une ligne de produit. Le modèle végétal selon l'invention permettra d'expliquer plus clairement les mécanismes à des non spécialistes du domaine concerné et par exemple de faire comprendre aux enfants pourquoi il ne faut pas trop s'exposer au soleil.
Une telle promotion du produit pourra se faire par n'importe quel canal de communication. Elle pourra être faite notamment par la vendeuse, directement sur le point de vente, par la radio et la télévision, notamment dans le cadre de spots publicitaires. Elle pourra être faite également par le canal de la presse écrite, ou par le biais de tout autre document, en particulier à des fins publicitaires (prospectus). Elle pourra se faire également par INTERNET, ou par tout autre réseau informatique adéquat. Elle pourra être faite également directement sur le produit, notamment sur son packaging ou sur toute notice explicative qui peut lui être associée.

Des visuels pourront être associés à une telle promotion, notamment sous forme de photographies, de vidéos, ou toute autre forme d'illustration, notamment des graphiques. De tels visuels seront représentatifs de l'effet du produit sur un ou plusieurs végétaux particuliers.

Selon encore un autre mode de réalisation, l'activité évaluée par le procédé selon l'invention est une activité indésirable, en particulier une toxicité ou un effet irritant du produit pour la peau, les muqueuses et/ou les phanères, voire même pour l'ensemble de l'organisme. L'invention a donc également pour objet un procédé de criblage pour identifier des effets indésirables d'actifs cosmétiques ou dermatologiques, comprenant les étapes suivantes:
a) évaluation de l'activité desdits actifs sur un marqueur biologique associé à un état normal par le procédé tel que défini précédemment,
b) comparaison des variations des paramètres mécaniques, optiques ou physico-chimiques induites par les actifs à cribler,
c) sélection des actifs ayant induits les variations les moins importantes à l'étape b)

Ce procédé pourra avantageusement se substituer au test de Ames

D'autres avantages de l'invention apparaîtront à la lecture des exemples qui suivent.

### Exemple 1: évaluation de l'effet hydratant

### I - MATÉRIEL ET MÉTHODE

### Modèle Végétal et Modèle Humain

1) Les plantes utilisées sont les suivantes :
   - Hydratation et Effet barrière :
      - Pommes de terres BF15 (origine maraîchers)
      - Carottes (origine maraîchers)
      - Pommes Golden (origine maraîchers)
      - Ficus (en pots à l'abri de la lumière solaire directe)
      - Syngonium (en pots à l'abri de la lumière solaire directe)
      - Spatyphyllum (en pots à l'abri de la lumière solaire directe)

   Les plantes ont été placées dans un environnement contrôlé afin de réaliser les mesures et tests.
2) Pour mesurer l'hydratation et l'effet barrière les modèles (2 volontaires Hommes et 2 volontaires Femmes) ont participé aux tests et mesures dans des conditions d'environnement également contrôlées.

### Actifs et Produits Cosmétiques utilisés :

- Hydratation et Effet barrière :
   - Vaseline
   - Solution à 3 % glycérol
   - Lauryl phosphate à 13 % (tensioactif)
   - Aquasource® - Biotherm -)
   - Effacil® - Lancôme -
   - Excell A3® - -

### Protocole de Mesures

Mesures réalisées autour de 22° C - Humidité relative entre 53 et 67 %.
- Applications sur modèle Végétal ou sur Humain :
   - Fruits et légumes : 2 échantillons par produit, mesurés à 24 heures d'intervalle. Application sur zones de 4cm². Une zone témoin sur chaque échantillon.
   - Plantes : 2 échantillons par produit, mesurés à 24 heures d'intervalle. Application des formules sur la totalité de la surface vernissée (supérieure). Une feuille par formule + une feuille témoin. Les feuilles choisies ont un aspect comparable.
   - Hommes : 4 modèles par produit. Application sur zone interne du bras. Trois zones de mesures dont une témoin, sur la partie interne de chaque avant bras. Les modèles sont placés au calme dans les conditions climatiques de mesure 15 minutes avant chaque série de mesures. Application de chaque formule sur une surface de 16 cm².
   - Application des formules à raison de 4mg/cm² (recherche du sens de l'action et non de la performance du produit).
- Mesures de l'hydratation et de l'effet barrière (modèle Végétal et Humain)
   - Hydratation - Mesure au cornéométre CM820 de la valeur moyenne de 5 déterminations.
   - Mesures à :
      . T0 : valeur de référence avant application des formules.
      . T2heures pour les actifs (mesure unique après constat d'apparition de coupures sur les feuilles provoquées par la pression de mesure du Cornéométre).
      . T1heure et T4heures pour les formules commercialisées.

### II - RÉSULTATS

### Résultats des mesures de l'hydratation (modèle Végétal et Humain)

Les valeurs représentent la moyenne des mesures à Ti divisées par celles à T0 corrigées des variations des zones témoins. L'accord entre l'homme et le végétal est obtenu par l'évolution comparable des mesures.

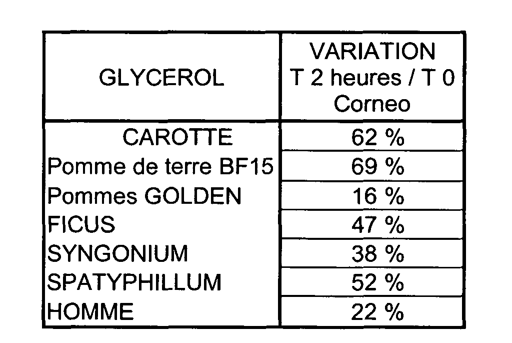

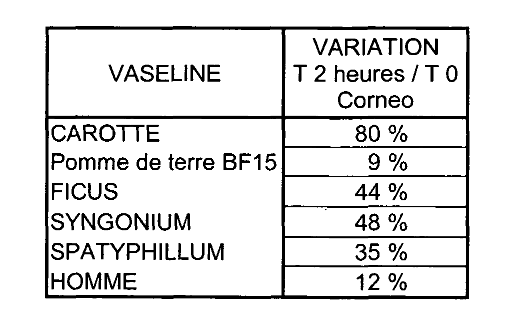

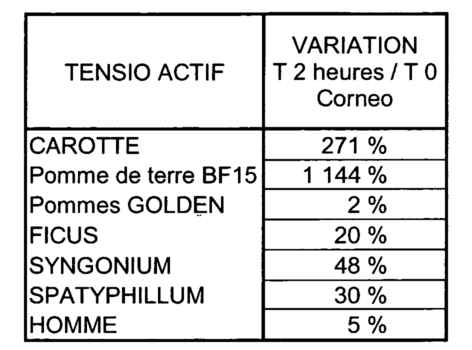

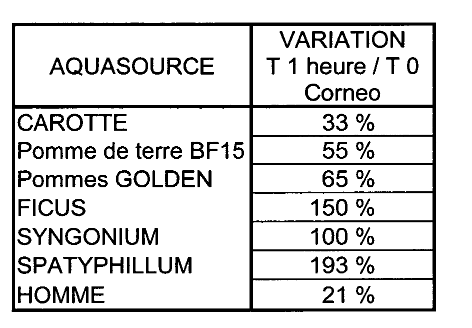

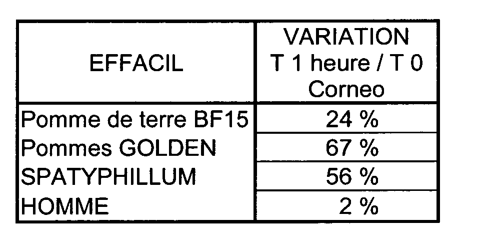

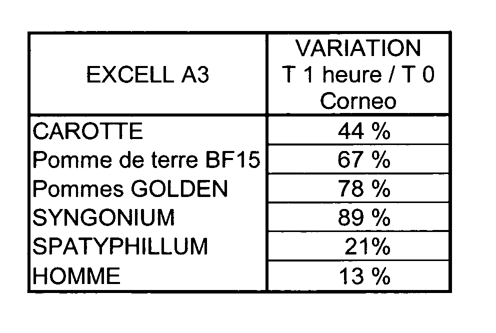

### Exemple 2: Evaluation de l'effet barrière:

Les modèles végétal et humain et les produits testés sont les mêmes que dans l'exemple 1
Les mesures de PIE (perte insensible en eau) sont réalisées selon le même protocole que précédemment
PIE : Mesure au Dermalab (sonde verticale au contact de la zone de mesure, sous pression minimale). Pour les plantes une plaque de verre est placée sous la zone de mesure. 2 séries de mesures consécutives de 25 valeurs.

Les valeurs représentent la moyenne des mesures à Ti divisées par celles à T0 corrigées des variations des zones témoins. L'accord entre l'homme et le végétal est obtenu par l'évolution comparable des mesures.

### Résultats:

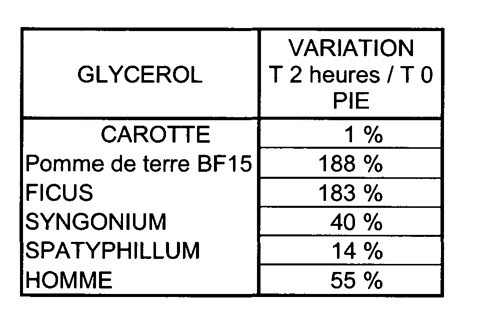

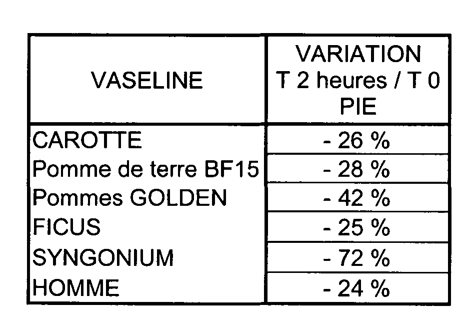

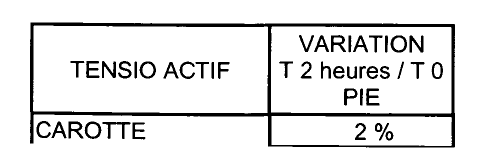

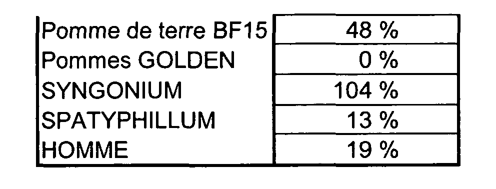

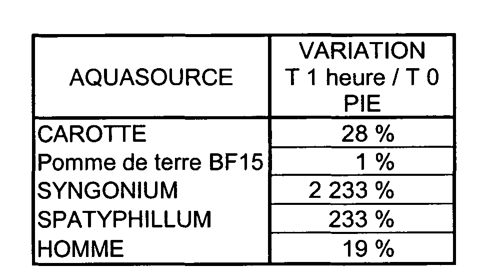

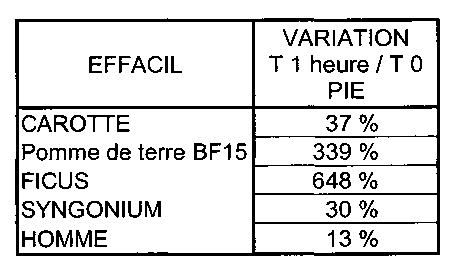

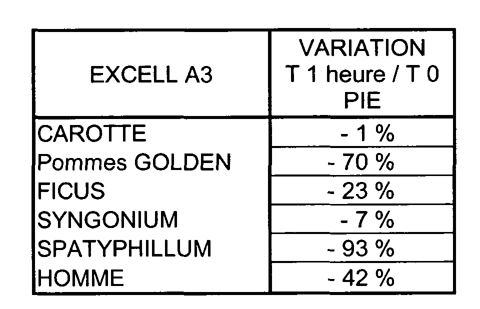

### Exemple 3: Evaluation d'un effet tenseur

Pour l'évaluation de l'Effet tenseur on utilise des Syngonium (en pots à l'abri de la lumière solaire directe)

Les plantes ont été placées dans un environnement contrôlé afin de réaliser les mesures et tests.

La composition T testée contient 7% d'une formule à base d'amidon de riz 7% et de copolymère acrylamide/acrylamido 2-ethyl propane sulfonate de sodium en émulsion inverse à 40 % dans un mélange isoparaffine/eau ).

Le protocole de mesure est le même que celui décrit dans l'exemple 1.

Les mesures de l'effet tenseur sont effectuées à l'extensomètre (in vitro) : Mesure selon la procédure MTT standard de l'effet tenseur de la composition. Les échantillons sont découpés dans des feuilles de syngonium. Les résultats sont comparés à ceux obtenus dans les mêmes conditions sur de la couche cornée humaine isolée (SC).

### Résultats des mesures de l'effet tenseur (in vitro)

| **MTT** | **SC HUMAIN ISOLE** | **SYNGONIUM** |
|---|---|---|
| **EFFET TENSEUR** | **-1,73** | **-2,58** |
| **INDICE DE CONFIANCE** (95%) | **0,33** | **0,32** |

**Observations :** le syngonium est deux fois plus sensible que le SC, avec une meilleure reproductibilité et une plus grande facilité d'utilisation.

### CONCLUSION

Le modèle végétal, dans les trois cibles d'activité que sont l'effet hydratant, l'effet barrière et les propriétés mécaniques, est un modèle intéressant présentant une dynamique plus forte que celle du modèle humain. Ainsi les valeurs au cornéomètre, de PIE, d'extensométrie évoluent-elles dans le même sens que sur l'homme.

## Revendications

1. Procédé d'évaluation de l'activité cosmétique ou dermatologique d'un produit **caractérisé en ce qu'**il comprend une étape consistant à mettre en contact le produit à évaluer avec un matériel végétal et à déterminer la variation d'un paramètre mécanique, optique ou physico-chimique dudit matériel végétal dans la zone de contact avec le produit, et/ou induite par le produit à évaluer.

2. Procédé selon la revendication 1 **caractérisé en ce que** l'activité cosmétique à évaluer est choisie parmi les effets sur l'état de surface, sur les secrétions, les réactions enzymatiques et sur les échanges gazeux.

3. Procédé selon la revendication 1 ou 2, **caractérisé en ce que** l'activité cosmétique évaluée pour le produit est choisie dans le groupe comprenant les effets sur l'hydratation de la peau, l'effet anti-rides, l'amélioration de la fonction barrière, la protection vis-à-vis des rayonnements solaires, l'amélioration des propriétés mécaniques de la peau, la desquamation, la pigmentation, l'effet tenseur, l'effet apaisant ou anti-irritant, l'effet d'interaction avec les odeurs, les propriétés couvrantes, les propriétés optiques colorantes ou masquantes et la tenue du produit.

4. Procédé selon l'une des revendications 1 à 3, **caractérisé en ce que** le produit à évaluer est mis en contact avec au moins une partie d'un végétal cultivé in vivo.

5. Procédé selon la revendication 4 **caractérisé en ce que** le produit à évaluer est appliqué sur, ou à proximité de, au moins une portion de la surface externe du végétal.

6. Procédé selon la revendication 4 **caractérisé en ce que** le produit à évaluer est mis en contact avec le végétal sous forme d'un nutriment qui est absorbé par ledit végétal.

7. Procédé selon l'une des revendications précédentes, **caractérisé en ce que** la variation du paramètre mécanique, optique ou physico-chimique est déterminée de manière qualitative.

8. Procédé selon l'une des revendications 1 à 6, **caractérisé en ce que** la variation du paramètre mécanique, optique ou physico-chimique est déterminée par des méthodes quantitatives.

9. Procédé selon l'une des revendications 1 à 8, **caractérisé en ce que** la variation du paramètre mécanique ou physico-chimique est déterminée par une méthode choisie parmi: cornéomètre, torquemètre, colorimètre, mesure du pH, sébumètre, lipomètre, microscope confocal, lumière blanche ou laser, desquamation, épaisseur de l'enveloppe cornéocytaire, microcirculation , perte insensible en eau, mesure du plissement cutané ( densiscore ), observation microscopique optique ou photonique, prise de photographie, mesure de spectres, analyse chromatographique, empreinte sur matériaux organique ou inorganique.

10. Procédé selon l'une des revendications 1 à 8 **caractérisé en ce que** le paramètre est un paramètre physico -chimique et que sa variation est mesurée par dosage enzymatique.

11. Procédé selon l'une des revendications précédentes **caractérisé en ce que** le matériel végétal est choisi parmi la plante entière, les fleurs, les feuilles, les tiges, les fruits, les graines, les racines, entier ou en fragment

12. Procédé de criblage pour identifier des actifs cosmétiques ou dermatologiques **caractérisé en ce que** l'on
a) évalue l'activité cosmétique ou dermatologique desdits actifs par le procédé selon l'une des revendications 1 à 11,
b) compare les variations des paramètres mécaniques, optiques ou physico-chimiques induites par les actifs à cribler,
c) sélectionne les actifs ayant induits les variations les plus importantes à l'étape b)

13. Procédé de préparation d'une composition cosmétique ou dermatologique, **caractérisé en ce que** l'on
i) sélectionne au moins un actif cosmétique ou dermatologique par le procédé selon la revendication 12,
ii) mélange le ou les actifs sélectionnés à l'étape i) avec des supports physiologiquement et/ou cosmétologiquement acceptables.

14. Utilisation d'au moins un matériel végétal ou d'un fragment de celui-ci pour évaluer l'activité cosmétique ou dermatologique d'un produit.
